# EUROPÄISCHE PATENTSCHRIFT

(11) **EP 2 468 703 B1**
(45) Veröffentlichungstag und Bekanntmachung des Hinweises auf die Patenterteilung: **15.04.2015**
(21) Anmeldenummer: 10196233.0
(22) Anmeldetag: 21.12.2010
(51) Int. Cl.: C07C 11/21, C07C 6/04, C08C 19/08

(54) **Verfahren zur Ethenolyse von Polyterpenen**
Method for ethenolysis of polyterpenes
Procédé d'éthénolyse de polyterpènes

(43) Veröffentlichungstag der Anmeldung: 27.06.2012
(73) Patentinhaber: Technische Universität Darmstadt, 64289 Darmstadt (DE)
(72) Erfinder: Plenio, Prof. Dr. Herbert, 64625, Bensheim (DE); Wolf, Dipl.-Ing. Stefanie, 67550, Worms (DE)
(74) Vertreter: Patentanwälte Olbricht Buchhold Keulertz

(56) Entgegenhaltungen:
- KENNETH B. WAGENER, RUTGER D. PUTS, DENNIS W. SMITH JR.: "Acyclic diene metathesis depolymerization of elastomers", DIE MAKROMOLEKULARE CHEMIE, RAPID COMMUNICATIONS, Bd. 12, Nr. 7, Juli 1991 (1991-07), Seiten 419-425, XP002639121, Basel ISSN: 1022-1336, DOI: 10.1002/marc.1991.030120708
- CRAIG S W ET AL: "Highly efficient acyclic diene metastasis depolymerization using a ruthenium catalyst containing a N-heterocyclic carbene ligand", MACROMOLECULES, AMERICAN CHEMICAL SOCIETY, US, Bd. 34, 6. November 2001 (2001-11-06), Seiten 7929-7931, XP002586803, ISSN: 0024-9297, DOI: DOI:10.1021/MA011188P [gefunden am 2001-10-04]
- LARS H. PEECK ET AL: "Synthesis and RCM Activity of [(NHC)(NHC ewg )RuCl 2 (3-phenylindenylid-1-ene)] Complexes", ORGANOMETALLICS, Bd. 29, Nr. 12, 28. Juni 2010 (2010-06-28) , Seiten 2761-2766, XP55000111, ISSN: 0276-7333, DOI: 10.1021/om1002717
- GUTIERRAS S ET AL: "Computational study of metathesis degradation of rubber. distributions of products for the ethenolysis of 1,4-polyisoprene", POLYMER DEGRADATION AND STABILITY, BARKING, GB, Bd. 83, Nr. 1, 1. Januar 2004 (2004-01-01) , Seiten 149-156, XP027166046, ISSN: 0141-3910 [gefunden am 2004-01-01]

## Beschreibung

Die vorliegende Erfindung betrifft die Gebiete organische Chemie, Organometallchemie und Polymerchemie.

### Stand der Technik

In den letzten Jahren ist das Interesse an der Entwicklung chemischer Verfahren zur Gewinnung von Chemikalien aus Biomasse enorm gestiegen. Aus diesem Grund wurden zahlreiche katalytische Reaktionen an Verbindungen aus Biomasse untersucht. Derzeit stellt die Umwandlung von Pflanzenölen, vor allem von Methyloleat oder Ethyloleat, das attraktivste Ziel für Olefinmetathesereaktionen dar. Zahlreiche Ansätze wie z.B. die Ethenolyse, die Kreuzmetathese und die Eninmetathese wurden mit Erfolg getestet.

Die Ethenolyse ist eine spezifische Variante der Olefin-Metathesereaktion zwischen einem internen Olefin und Ethen, mit der terminale Olefine gewonnen werden.

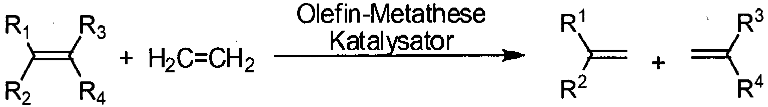

Die Ethenolyse von Pflanzenölen ist ein Beispiel für die Ethenolyse zweifach alkylierter Doppelbindungen. In der Regel wird hierbei Ölsäure eingesetzt, meist als entsprechender Ester. Olefin-Metathesereaktionen höher substituierter (dreifach oder vierfach alkylierter) Doppelbindungen sind erheblich erschwert, da die räumlich anspruchsvolleren Substituenten die Ethenolyse erschweren. Unter einer dreifachsubstituierten Doppelbindung versteht man eine C=C Doppelbindung, an die drei Nichtwasserstoffatome gebunden sind, typischerweise handelt es sich um eine Alkylgruppe CR₃, wobei es sich um R = H oder R = C oder beliebige andere Atome handeln kann.

Die US 2010 / 0022789 A1 beschreibt katalytische Zusammensetzungen für die Metathese ungesättigter Fettsäuren mit Olefinen und entsprechende Metatheseverfahren. Die katalytische Zusammensetzung umfasst dabei einen Ruthenium-Carben-Komplex vom Typ der Grubbs-Hoveyda-Katalysatoren. Die Herstellung der erfindungsgemäßen Katalysatorzusammensetzungen sowie die Metathese von Methyloleat sind offenbart. Es sind jedoch keine Metathesereaktionen an dreifach substituierten Doppelbindungen beschrieben.

Die WO 2008 / 010961 A1 beschreibt die Herstellung terminaler Alkene aus internen Alkenen und Ethen mittels Olefin-Metathese. Dabei werden Ruthenium- und Osmium-Katalysatoren des Grubbs II- und des Grubbs-Hoveyda-Typs eingesetzt. In den bevorzugten Ausführungsformen ist das interne Olefin ein Glycerid und/oder bei der Ethenolyse wird ein Keimöl zugegeben. Die Ausführungsbeispiele betreffen ausschließlich die Ethenolyse von Glyceriden bzw. Reaktionen in Gegenwart von Keimölen. Exemplarisch ist die Ethenolyse von Methyloleat zu Methyl-9-decenoat gezeigt.
Die WO 2008 / 046106 A2 beschreibt ebenfalls die Herstellung terminaler Alkene aus internen Alkenen mittels Olefin-Metathese. Es werden Grubbs II- und Grubbs-Hoveyda-Katalysatoren mit Ruthenium als Zentralatom eingesetzt. Beim erfindungsgemäßen Verfahren werden ein olefinisches Substrat umfassend mindestens ein internes Olefin mit einem Kreuzmetathesepartner umfassend einen alpha-olefinischen Reaktanden in Gegenwart eines Ruthenium-Alkyliden-Metathesekatalysators unter Reaktionsbedingungen umgesetzt, die eine Kreuzmetathesereaktion gestatten, wobei die Reaktionstemperatur mindestens 35 °C beträgt. Bevorzugt handelt es sich bei den internen Olefinen um Glyceride, die optional ein (Keim-)Öl umfassen. Die Ausführungsbeispiele betreffen ausschließlich Fettsäuremethylester; Reaktionsprodukte der Ethenolyse sind 1-Decen, 2-Undecen, 3-Dodecen, Methyl-9-decenoat und Methyl-9-dodecenoat. Eine Ethenolyse von Olefinen mit dreifach substituierten Doppelbindungen ist weder in dieser Schrift noch in der WO 2008 / 010961 A1 gezeigt.
Bei beiden genannten Schriften, d.h. bei WO 2008 / 010961 A1 und bei WO 2008 / 046106 A2 ist der Ruthenium-Carben-Komplex ein sog. NHC-Komplex, d.h. ein Komplex eines *N*-heterocyclischen Carbens. Bei keiner der beiden Schriften führt die Ethenolyse zu Abbauprodukten, die interne Olefine bzw. mehr als eine Kohlenstoff-Kohlenstoff-Doppelbindung enthalten.

Einige der natürlich vorkommenden Verbindungen mit dreifach substituierten Doppelbindungen gehören zur Gruppe der Terpene. Die Terpene sind eine stark heterogene und sehr große Gruppe von chemischen Verbindungen, welche als sekundäre Inhaltsstoffe in Organismen natürlich vorkommen. Sie leiten sich formal vom Isopren ab und zeichnen sich dabei durch eine große Vielfalt an Kohlenstoffgerüsten und geringer Anzahl an funktionellen Gruppen aus. Die überwiegende Mehrzahl aller Terpene enthält dreifach substituierte Doppelbindungen. Es sind über 30.000 Terpene bekannt. Die meisten Terpene sind Naturstoffe, hauptsächlich pflanzlicher und seltener tierischer Herkunft. In der Natur kommen überwiegend Kohlenwasserstoff-, Alkohol-, Glycosid-, Ether-, Aldehyd-, Keton-, Carbonsäure- und Ester-Terpene vor, aber auch Vertreter weiterer Stoffgruppen sind unter den Terpenen zu finden. Die Terpene sind Hauptbestandteil der in Pflanzen produzierten ätherischen Öle.
Die Terpene kann man in Isopreneinheiten unterteilen, die die gleiche Anzahl von Kohlenstoffatomen haben. Jede Isopreneinheit umfasst fünf Kohlenstoffatome. Terpene mit 5 Kohlenstoffatomen nennt man Hemiterpene (C₅), mit zehn Monoterpene (C₁₀), mit 15 Sesquiterpene (C₁₅), mit 20 Diterpene (C₂₀), mit 25 Sesterterpene (C₂₅), mit 30 Triterpene (C₃₀) und mit 40 Tetraterpene (C₄₀). Terpene mit mehr als 8 Isopreneinheiten, also mit mehr als 40 Kohlenstoffatomen, nennt man Polyterpene (größer als C₄₀).
Man unterscheidet zudem, ob die Isopreneinheiten *Kopf-Schwanz*, *Kopf-Kopf* oder *Schwanz-Schwanz* verbunden sind. Dies wird "Biogenetische Isoprenregel" genannt . Die Seite der Isopreneinheit, welche die Isopropylgruppe enthält, wird *Kopf* genannt, das Ende der Isopropyleinheit, welches unsubstituiert ist, wird *Schwanz*, im Sinne von Verknüpfungen der Einheiten zu längeren Bausteinen, genannt.

Wichtige natürlich vorkommende Terpene sind beispielsweise Naturkautschuk (ein Polyterpen) sowie Squalen (ein Triterpen). Naturkautschuk und Squalen enthalten dreifach substituierte Doppelbindungen.

Naturkautschuk ist eine Klasse elastischer Polymere, die vorzugsweise aus dem Milchsaft (Latex) oder den Blättern verschiedener tropischer Pflanzen gewonnen werden. Die wichtigste Quelle für Naturkautschuk ist der Kautschukbaum (Hevea brasiliensis), daneben auch beispielsweise der Balatabaum, der Breiapfelbaum, der Guttaperchabaum oder die Guayulepflanze. Ein besonderes Merkmal von Hevea brasiliensis-Naturkautschuk, welches diesen von synthetischem Kautschuk (Polyisopren) unterscheidet, ist die hohe Stereoregularität (> 99,5 % cis-Doppelbindungen) des natürlichen Materials. Eine Variante des Naturkautschuks, Guttapercha, ist ebenfalls stereoregulär, enthält aber primär trans-Doppelbindungen. Dies bedeutet, dass Abbaureaktionen (Ethenolyse) insbesondere für Naturkautschuk besonders nützlich sind, weil eine geringe Zahl stereochemisch genau definierter Produkte erwartet werden kann. Die Molekularmasse von Hevea Brasiliensis-Naturkautschuk bewegt sich typischerweise im Bereich von 100.000 - 1.000.000 g/mol. Wegen seines hohen Grades an Stereoregularität im Hinblick auf die Geometrie der Doppelbindungen und der Kopf-Schwanz-Orientierung sollte Naturkautschuk eine wertvollere Quelle für Ethenolysereaktionen darstellen als synthetisch hergestellter Polyisopren-Kautschuk. Bei der Ethenolyse synthetisch hergestellten Polyisopren-Kautschuks kommt es aufgrund der im Vergleich zu Naturkautschuk signifikant geringeren Stereoregularität unweigerlich zur Bildung zahlreicher isomerer Olefine. Die Ethenolyse von Naturkautschuk, d.h. von natürlichem cis-1,4-Polyisopren und von natürlichem trans-1,4-Polyisopren (Guttapercha), sollte wegen seiner hohen Stereoregularität zu einer deutlich geringeren Zahl von unterschiedlichen Abbauprodukten und weniger Isomeren führen. Im Stand der Technik ist eine derartige Ethenolyse mit wenigen, definierten Abbauprodukten bislang allerdings unbekannt.

Squalen ist ein Triterpen, enthält also sechs Isopreneinheiten. Je drei dieser Isopreneinheiten sind zu Sesquiterpenen mit Kopf-Schwanz-Orientierung verknüpft, und die beiden Sesquiterpeneinheiten sind in Schwanz-Schwanz-Orientierung miteinander verbunden. Squalen wird von allen höheren pflanzlichen und tierischen Organismen produziert. Es spielt beispielsweise bei der Biosynthese von Cholesterin, Steroidhormonen und Vitamin D eine wichtige Rolle und ist ein wesentlicher Bestandteil der Hautlipide. Die Aufarbeitung verschiedener Öle, insbesondere von Olivenöl und Haifischleberöl, stellt die wichtigste Quelle für Squalen dar.

Terpene sind vielfach biologisch und pharmakologisch interessant. Sie können als umweltfreundliche Insektizide verwendet werden, indem sie als Pheromone Insekten in Fallen locken. Außerdem wirken viele antimikrobiell. Viele Terpene werden als Geruchs- oder Geschmacksstoffe in Parfums und kosmetischen Produkten eingesetzt. Bislang ist jedoch nur ein Bruchteil der theoretisch denkbaren Terpene tatsächlich existent und charakterisiert.

In A Alimuniar, MA Yarmo, MZ Ab.Rahman, S Kohjiya, Y Ikeda, S Yamashita: "Metathesis degradation of natural rubber", Polym Bull 1990, 23, 119-126 und in KB Wagener, RD Puts, DW Smith jr.: "Acyclic diene metathesis depolymerization of elastomers", Macromol Chem Rapid Commun 1991, 12, 419-425 wird der teilweise Abbau von Naturkautschuk in Gegenwart von Wolfram-basierten Katalysatoren und Ethen beschrieben, wobei nicht genau bestimmbare Gemische entstehen.

In Guiterras et al: "Computational study of metathesis degradation of rubber. distibutions of products for the ethenolysis of 1,4-polyisoprene" Polymer Degradation and Stability 2004, 83, 149- 156 ist eine theoretische Studie zur Gleichgewichtslage bei der Ethenolyse von cis-1,4-Polyisopren (natural rubber) und trans-1,4-Polyisopren beschrieben. Es findet sich jedoch kein Hinweis darauf, wie eine effiziente Ethenolyse praktisch durchgeführt werden kann.

Andere bekannte Polymerethenolysen sind bisher auf disubstituierte Olefine oder Polyisoprene in verschiedenen Gummimischungen beschränkt. Dies ist beispielweise in SW Craig, JA Manzer, EB Coughlin: "Highly Efficient Acyclic Diene Metathesis Depolymerization Using a Ruthenium Catalyst Containing a N-Heterocyclic Carbene Ligand", Macromolecules 2001, 34, 7929-7931 beschrieben. Der bislang nur geringe Erfolg bei der Ethenolyse von Naturkautschuk scheint auf die geringere Reaktivität der dreifach alkylierten Doppelbindungen im Vergleich zu den zweifach substituierten in Ölsäure zurückzuführen zu sein, während die sekundären Metathesereaktionen der bei der Ethenolyse entstehenden terminalen Olefine offenbar nicht gehemmt wird. Dies ist in S Fomine, MA Tlenkopatchev: "Organometallic Computational Modeling of Renewable Molecules. Ruthenium Alkylidene-Mediated Metathesis of Trialkyl-Substituted Olefins", Organometallics 2010, 29, 1580-1587 beschrieben.

Eine neue Generation von Katalysatoren, die den Grubbs- und Grubbs-Hoveyda-Katalysatoren strukturell ähneln, sind die Verbindungen [(NHC)(NHC_{ewg})RuCl₂(CHPh)]. Dabei steht NHC für eine vergleichsweise elektronenreiche *N*-heterocyclische Carbengruppierung, und NHC_{ewg} repräsentiert eine relativ elektronenarme *N*-heterocyclische Carbengruppierung. "Relativ elektronenreich" bzw. "relativ elektronenarm" bezieht sich dabei auf die jeweils andere NHC-Gruppierung: Der NHC-Ligand ist also regelmäßig elektronenreicher als der NHC_{ewg}-Ligand. Der Index "ewg" bedeutet im Allgemeinen "electron withdrawing group", d.h. "elektronenziehende Gruppe". Im Falle der Grubbs- und Grubbs-Hoveyda-Katalysatoren wird der Index "ewg" jedoch vor allem verwendet, um den üblicherweise elektronenärmeren NHC-Liganden zu kennzeichnen. Dem Fachmann ist bekannt, dass Elektronenreichtum bzw. Elektronenarmut einer chemischen Struktur von Polarisationseffekten der beteiligten Atome, Substituenten und funktionellen Gruppen abhängen. Für ein Reaktandmolekül RY umfasst der Polarisationseffekt der Gruppe R alle Vorgänge, durch die ein Substituent die elektrostatischen Kräfte verändert, die am Reaktionszentrum Y wirksam sind, und zwar im Vergleich zu einem Standard R⁰Y. Diese Kräfte können beeinflusst werden
a) von Ladungstrennungen, die durch unterschiedliche Elektronegativitäten der beteiligten Atome zustande kommen (wodurch Dipole vorliegen),
b) durch das Vorliegen von Unipolen oder
c) durch Elektronendelokalisation.
Bei den Verbindungen [(NHC)(NHC_{ewg})RuCl₂(CHPh)] stellen NHC und NHC_{ewg} Reaktandmoleküle gemäß obiger Definition dar. Dabei üben die Gruppen R der relativ elektronenarmen NHC_{ewg}-Gruppierung einen stärker positiv polarisierenden Effekt auf das Reaktionszentrum dieser Gruppierung aus, als es die Reaktanden R der relativ elektronenreichen NHC-Gruppierung mit deren Reaktionszentrum tun.

In T Vorfalt, S Leuthäußer, H Plenio: "An [(NHC)(NHCewg)RuCl2(CHPh)] Complex for the Efficient Formation of Sterically Hindered Olefins by Ring-Closing Metathesis", Angew Chem Int Ed 2009, 48, 5191-5194 und in V Sashuk, LH Peeck, H Plenio: "[(NHC)(NHCewg)RuCl2(CHPh)] Complexes with Modified NHCewg Ligands for Efficient Ring-Closing Metathesis Leading to Tetrasubstituted Olefins", Chem Eur J 2010, 16, 3983-3993 ist die Herstellung solcher Katalysatoren sowie deren Verwendung für die Ringschlussmetathese von 1,ω-Dienen zu tetrasubstituierten Cycloolefinen beschrieben. In der Publikation von Sashuk et al. wird darauf hingewiesen, dass der NHC_{ewg}-Ligand, d.h. der elektronenarme Ligand, in aller Regel als Abgangsgruppe fungiert, deren Abspaltung zur Aktivierung des Katalysators führt.
In LH Peeck, H Plenio: "Synthesis and RCM Activity of [(NHC)(NHCewg)RuCl2(3-phenylindenylid-1-ene)] Complexes", Organometallics 2010, 29, 2761-2766 und in S Wolf, H Plenio: "Facile synthesis of [(NHC)(NHCewg)RuCl2(CHPh)] complexes", J Org Chem 2010, 695, 2418-2422 werden die Synthese weiterer NHC-NHC_{ewg}-Komplexe des Rutheniums sowie deren Verwendung für die Ringschlussmetathese offenbart.

Bislang kennt der Stand der Technik jedoch kein Verfahren, dass die Ethenolyse von Polyterpenen mit dreifach substituierten Doppelbindungen erlaubt.

### Aufgabe

Aufgabe der Erfindung ist es, ein Verfahren zur Ethenolyse von acyclischen Polyterpenen, die dreifach substituierte Doppelbindungen umfassen.

### Lösung der Aufgabe

Die Aufgabe, ein Verfahren zur Ethenolyse von acyclischen Polyterpenen, die dreifach substituierte Doppelbindungen umfassen, bereitzustellen, wird erfindungsgemäß gelöst durch ein Verfahren umfassend die Schritte:
a) Inkontaktbringen eines Polyterpens mit einem Katalysatorkomplex ausgewählt aus [(NHC)(NHC_{ewg})MeX₂(CHPh)] und [(NHC)(NHC_{ewg})MeX₂(3-phenylindenylid-1-en)] in einem druckfesten Reaktor, worin
   - NHC und NHC_{ewg} für *N*-heterocyclische Carbenliganden stehen, wobei NHC elektronenreicher als NHC_{ewg} ist,
   - Me ausgewählt ist aus Ru und Os,
   - X ausgewählt ist aus F, Cl, Br, I, CN, SCN, OCN, NCO,
b) Spülen des Reaktors mit Schutzgas,
c) Spülen des Reaktors mit Ethen,
d) Reaktion des Gemisches aus Polyterpen und Katalysatorkomplex mit Ethen bei einem Ethendruck von 1 bis 100 bar und einer Temperatur von 60 °C bis 150 °C für 15 min bis 24 h,
e) Beenden der Ethenzugabe, Abkühlen auf Raumtemperatur und ggf. Entspannen auf Umgebungsdruck,
f) optional Auftrennen der erhaltenen Spaltprodukte.

Überraschend wurde gefunden, dass Polyterpene, die dreifach substituierte acyclische Doppelbindungen umfassen, mittels Ethenolyse abbaubar sind, sofern die Ethenolyse in Gegenwart der oben beschriebenen Katalysatoren und unter den oben aufgeführten Reaktionsbedingungen stattfindet. Das erfindungsgemäße Verfahren ermöglicht die weitgehende Unterdrückung der sekundären Metathesereaktion der Vinylendgruppen und liefert im Gegensatz zu bisher bekannten Ethenolysereaktionen von Terpenen eine vergleichsweise kleine Anzahl von Abbauprodukten.

Das erfindungsgemäße Verfahren, die daraus erhältlichen Abbauprodukte sowie deren Verwendung sind nachfolgend erläutert.

Die Erfindung ist nicht auf eine der nachfolgend beschriebenen Ausführungsformen beschränkt, sondern in vielfältiger Weise abwandelbar.

Sämtliche aus den Ansprüchen und der Beschreibung hervorgehenden Merkmale und Vorteile, einschließlich konstruktiver Einzelheiten, räumlicher Anordnungen und Verfahrensschritten, können sowohl für sich als auch in den verschiedensten Kombinationen erfindungswesentlich sein.

Im erfindungsgemäßen Verfahren werden Katalysatorkomplexe ausgewählt aus [(NHC)(NHC_{ewg})MeX₂(CHPh)] und [(NHC)(NHC_{ewg})MeX₂(3-phenylindenylid-1-en)] eingesetzt, worin Ph für eine Phenylgruppe steht. Dabei stehen NHC und NHC_{ewg} für *N*-heterocyclische Carbenliganden, wobei NHC elektronenreicher als NHC_{ewg} ist. Elektronenschiebende Gruppen sind dabei funktionelle Gruppen mit einem positiven induktiven und/oder positiven mesomeren Effekt (+I-Effekt, +M-Effekt). Derartige Substituenten erhöhen die Elektronendichte der Nachbaratome über eine oder mehrere chemische Bindungen, wobei die Erhöhung der Elektronendichte der Nachbaratome mit steigender Distanz vom elektronenschiebenden Substituenten abnimmt. Atome oder Atomgruppen, deren Elektronendichte durch elektronenschiebende Substituenten erhöht ist, nennt man "elektronenreich". Analog sind elektronenziehende Gruppen funktionelle Gruppen mit einem negativen induktiven und/oder negativen mesomeren Effekt (-I-Effekt, -M-Effekt). Sie verringern die Elektronendichte der Nachbaratome über eine oder mehrere chemische Bindungen, und auch der elektronenziehende Effekt auf die Nachbaratome nimmt mit steigender Distanz vom elektronenziehenden Substituenten ab. Während allen Substituenten (außer dem Wasserstoff-Atom als Referenzgruppe) ein induktiver Effekt zugeschrieben werden kann, liegt ein mesomerer Effekt nicht zwingend vor. Der mesomere Effekt eines Substituenten kann in die gleiche Richtung weisen wie sein induktiver Effekt, die Effekte können aber auch gegenläufig sein. Trägt eine chemische Verbindung mehrere funktionelle Gruppen, so hängen Elektronenreichtum bzw. Elektronenarmut dieser Verbindung vom Gesamteinfluss aller Substituenten ab.
Die Veränderung der Elektronendichte eines Atoms durch induktive oder mesomere Effekte von Nachbarsubstituenten bezeichnet man auch als Polarisation.
Tabellenwerke, in denen die Stärke der induktiven und - so verhanden - mesomeren Effekte verschiedener Substituenten aufgeführt sind, sind dem Fachmann bekannt. "NHC ist mindestens so elektronenreich wie NHC_{ewg}" bedeutet erfindungsgemäß, dass Elektronenreichtum bzw. Elektronenarmut der beiden N-heterocyclischen Carbene relativ zueinander zu betrachten sind: Die Elektronendichte der Gruppierung N-C-N ist im Liganden NHC mindestens so hoch wie im Liganden NHC_{ewg}, und zwar unabhängig von absoluten Zahlenwerten der Elektronendichte. Es ist auch möglich, dass der Ligand NHC_{ewg} gar keinen elektronenziehenden Substituenten trägt, denn solange der Ligand NHC elektronenreicher ist, ist NHC_{ewg} der elektronenärmere Carbenligand gemäß obiger Definition.
Dabei ist NHC elektronenreicher als NHC_{ewg}.

In den erfindungsgemäß einzusetzenden Katalysatorkomplexen ist das Metall Me ausgewählt aus Ru und Os. Der Ligand X ist ein Halogenid oder Pseudohalogenid, ausgewählt aus F, Cl, Br, I, CN, SCN, OCN, NCO.

In einer vorteilhaften Ausführungsform handelt es sich bei den erfindungsgemäß einzusetzenden Katalysatorkomplexen um Verbindungen der Formel (I): 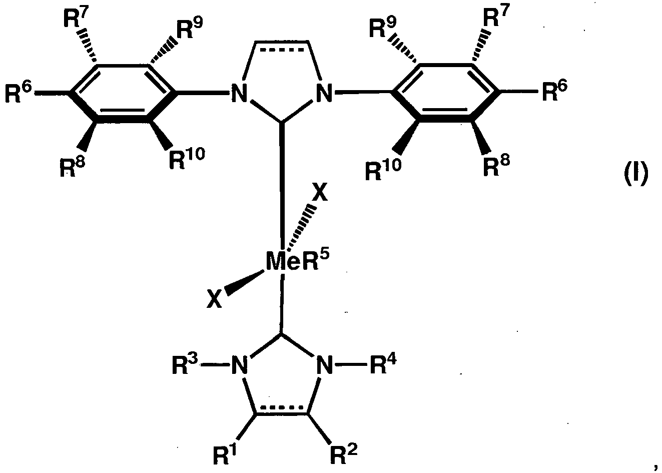 worin
- R¹ und R²: unabhängig voneinander für -H, -F, -Cl, -Br, -I, -CN, -SCN, -OCN, -NCO, -NO₂ oder eine lineare, verzweigte oder cyclische Alkylgruppe mit 1 bis 20 Kohlenstoffatomen, stehen,
- R³ und R⁴: unabhängig voneinander für -H, eine lineare, verzweigte oder cyclische Alkylgruppe mit 1 bis 20 Kohlenstoffatomen, oder eine Gruppe ausgewählt aus 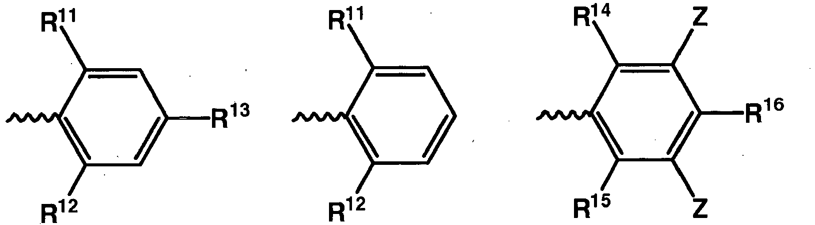 stehen, worin
- R¹¹, R¹² und R¹³: unabhängig voneinander für -H, -F, -Cl, -Br, -I, -CN, -SCN, -OCN, -NCO, -NO₂ oder eine lineare oder verzweigte Alkylgruppe mit 1 bis 20 Kohlenstoffatomen stehen,
- R¹⁴, R¹⁵ und R¹⁶: unabhängig voneinander für -H oder eine lineare oder verzweigte Alkylgruppe mit 1 bis 20 Kohlenstoffatomen stehen
und
Z für -NO₂ oder -SO₃H stehen,
R⁵ für eine Gruppe 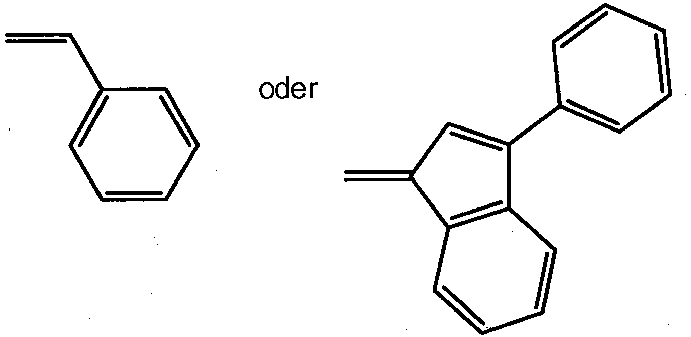 steht,
- R⁶: für -H oder eine lineare, verzweigte oder cyclische Alkylgruppe mit 1 bis 20 Kohlenstoffatomen und
- R⁷ und R⁸: unabhängig voneinander für -H, -F, -Cl, -Br, -I, -CN, -SCN, -OCN, -NCO, -NO₂, -OH, -SH, -NH₂, -OCH₃ oder eine lineare, verzweigte oder cyclische Alkylgruppe mit 1 bis 20 Kohlenstoffatomen und
- R⁹ und R¹⁰: unabhängig voneinander für eine lineare oder verzweigte Alkylgruppe mit 1 bis 4 Kohlenstoffatomen stehen und
Me für Ru oder Os steht,
X für ein Halogen oder Pseudohalogen steht, ausgewählt aus -F, -Cl, -Br, -I, -CN, -SCN, -OCN, -NCO und 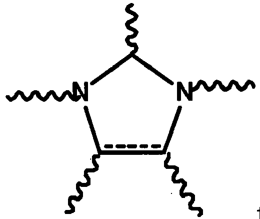 für einen Imidazolidin- oder 2,3-Dihydroimidazolrest steht.

Die Gruppierung 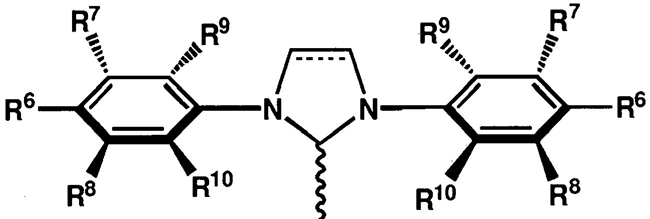 stellt dabei den NHC-Liganden und die Gruppierung 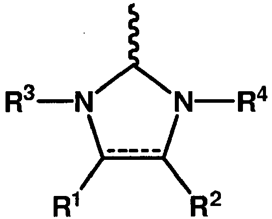 den NHC_{ewg}-Liganden dar.
Die gestrichelte Linie zwischen den Kohlenstoffatomen 4 und 5 des NHC_{ewg}-Liganden bedeutet dabei, dass diese beiden Kohlenstoffatome über eine C-C-Einfachbindung (Imidazolidinrest) oder eine C=C-Doppelbindung (2,3-Dihydroimidazolrest) miteinander verbunden sind.

Der erfindungsgemäß einzusetzende Katalysatorkomplex wird während des erfindungsgemäßen Verfahrens aktiv, indem die NHC_{ewg}-Gruppe abdissoziiert. Dabei handelt es sich um die Gruppierung 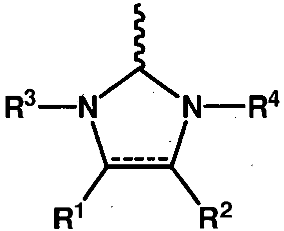 der erfindungsgemäß einzusetzenden Katalysatorkomplexe.
Der Rest des Katalysatorkomplexes, der nach der Abspaltung der NHC_{ewg}-Gruppe übrig bleibt, ist die katalytisch aktive Spezies. Es konnte gezeigt werden, dass das erfindungsgemäße Verfahren umso besser funktioniert, je langsamer die NHC_{ewg}-Gruppe abgespalten wird Derartige Katalysatoren sind dem Fachmann auch als sogenannte latente Katalysatoren bekannt (S. Monsaert, A. L. Vila, R. Drozdzak, P. V. D. Voort, F. Verpoort, Chem. Soc. Rev. 2009, 38, 3360-3372). Diese Katalysatoren zeichnen sich bei Raumtemperatur durch eine kaum vorhandene katalytische Aktivität aus und werden erst z. B. durch Temperaturerhöhung aktiviert.

Das erfindungsgemäße Verfahren eignet sich besonders für die Ethenolyse zum Abbau natürlich vorkommender acyclischer Polyterpene mit dreifach substituierten Doppelbindungen mittels Ethen durch Olefin-Metathesekatalysatoren. Es ist aber auch für andere acyclische Terpene mit dreifach substituierten Doppelbindungen geeignet. Natürlich vorkommende Polyterpene mit dreifach substituierten Doppelbindungen sind insbesondere Naturkautschuk und Squalen.
Der Stand der Technik kennt bislang kein Verfahren, das den Abbau von Polyterpenen mit dreifach substituierten acyclischen Doppelbindungen zu Verbindungen gemäß Anspruch 10 ermöglicht.

In einer bevorzugten Ausführungsform der vorliegenden Erfindung ist das Polyterpen umfassend dreifach substituierte acyclische Doppelbindungen ausgewählt aus Naturkautschuk, Squalen und Liquid Natural Rubber. Squalen besitzt die Summenformel C₃₀H₅₀ und ist damit ein Triterpen, während Polyterpene gemäß der allgemein üblichen Definition mindestens 40 Kohlenstoffatome umfassen. Im Rahmen der vorliegenden Erfindung umfasst der Begriff "Polyterpen" jedoch auch Squalen. Der Begriff "Naturkautschuk" umfasst dabei sowohl natürliches cis-1,4-Polyisopren als auch natürliches trans-1,4-Polyisopren (Guttapercha). Liquid Natural Rubber wird industriell hergestellt durch den kontrollierten Abbau von Naturkautschuk mit chemischen Reagenzien (z.B. Phenylhydrazin in Gegenwart von Luftsauerstoff). Dabei enstehen Kautschukmoleküle mit verkürzter Kettenlänge bzw. verringerter Molekularmasse. Typische kommerziell verfügbare Produkte weisen Molekularmassen im Bereich von 30.000 bis 60.000 g/mol auf.

Im Rahmen der vorliegenden Erfindung werden Alkylgruppen mit 1 bis 20 Kohlenstoffatomen ausgewählt aus Methyl, Ethyl, n-Propyl, Isopropyl, 1-Butyl, 2-Butyl, tert.-Butyl, 1-Pentyl, 2-Pentyl, 3-Pentyl, 3-Methylbutyl, 2,2-Dimethylpropyl sowie allen Isomeren von Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl und Eicosyl.

Dem Fachmann ist bekannt, dass cyclische Alkyl- und Alkenylgruppen mindestens drei Kohlenstoffatome enthalten müssen. Im Rahmen der vorliegenden Erfindung werden unter "ringförmigen" Gruppen solche Gruppen verstanden, bei denen alle Kohlenstoffatome an der Ringbildung beteiligt sind. "Cyclische" Gruppen können darüber hinaus optional noch acyclische Kohlenstoffatome enthalten. Ringförmige Alkylgruppen im Rahmen der vorliegenden Erfindung sind Propyl-, Butyl-, Pentyl-, Hexyl-, Heptyl-, Octyl-, Nonyl-, Decyl-, Undecenyl-, Dodecenyl-, Tridecenyl-, Tetradecenyl-, Pentadecenyl-, Hexadecenyl-, Heptadecenyl-, Octadecenyl-, Nonadecenyl- und Eicosylringe. Handelt es sich bei den Gruppen R¹, R², R³, R⁴, R⁶, R⁷ und R⁸ um cyclische Alkylgruppen, so werden diese ausgewählt aus den genannten ringförmigen Alkylgruppen, die keine weiteren Substituenten tragen, und aus den genannten ringförmigen Alkylgruppen, welche ihrerseits an eine oder mehrere acyclische Alkylgruppen gebunden sind. Im letztgenannten Fall kann die Bindung der cyclischen Alkylgruppe an die Phenylgruppe (im Falle von R⁶) bzw. an ein Stickstoff- oder Kohlenstoffatom des Imidazolidin- oder 2,3-Dihydroimidazolrings (im Falle von R¹, R², R³ und R⁴) über ein cyclisches oder ein acyclisches Kohlenstoffatom der cyclischen Alkyl- oder Alkylengruppe erfolgen. Cyclische Alkylgruppen enthalten gemäß obiger Definition des Begriffes "Alkylgruppe" ebenfalls insgesamt maximal 20 Kohlenstoffatome.

Handelt es sich bei R⁹ und R¹⁰ um eine lineare oder verzweigte Alkylgruppe mit 1 bis 4 Kohlenstoffatomen, so ist diese ausgewählt aus Methyl, Ethyl, 1-Propyl, 2-Propyl, 1-Butyl, 2-Butyl und tert.-Butyl.

Das Zentralatom Me des erfindungsgemäß einzusetzenden Katalysatorkomplexes ist ausgewählt aus Ruthenium und Osmium. In einer bevorzugten Ausführungsform handelt es sich bei dem Zentralatom Me um Ruthenium.

In einer weiteren vorteilhaften Ausführungsform ist der Rest R⁶ des Katalysatorkomplexes ein Wasserstoffatom oder eine Methylgruppe, die Reste R⁷ und R⁸ sind Wasserstoffatome und die Reste R⁹ und R¹⁰ stellen Methylgruppen dar.

In einer weiteren bevorzugten Ausführungsform sind die Reste R¹ und R² unabhängig voneinander ausgewählt aus -H, -Cl, -NO₂, -CN und 3,5-Dinitro-2,4,6-trimethylphenyl.

In einer weiteren bevorzugten Ausführungsform sind die Reste R³ und R⁴ sind unabhängig voneinander ausgewählt aus Methyl-, Ethyl- und Isopropylgruppen.

Besonders bevorzugt sind die Reste R¹ und R² unabhängig voneinander ausgewählt aus -H, -Cl, -NO₂, -CN und 3,5-Dinitro-2,4,6-trimethylphenyl, und die Reste R³ und R⁴ sind unabhängig voneinander ausgewählt aus Methyl-, Ethyl- und Isopropylgruppen.

Gemäß Schritt a) des erfindungsgemäßen Verfahrens werden das Polyterpen und der Katalysatorkomplex in einem druckfesten Reaktor miteinander in Kontakt gebracht. Dabei kann das Terpen im Reaktor vorgelegt und anschließend der Katalysator zugefügt werden oder umgekehrt, oder beide können gleichzeitig zugegeben werden. Bei gleichzeitiger Zugabe von Terpen und Katalysator in den druckfesten Reaktor kann die Reaktionsführung kontinuierlich oder diskontinuierlich erfolgen. Die kontinuierliche Reaktionsführung kann vorteilhaft in einem Membranreaktor durchgeführt werden.
In einer Ausführungsform des erfindungsgemäßen Verfahrens werden Polyterpen und Katalysatorkomplex ohne Zusatz eines Lösungsmittels miteinander in Kontakt gebracht und anschließend gemäß den Schritten b) bis f) des erfindungsgemäßen Verfahrens weiter verarbeitet..
In einer weiteren Ausführungsform des erfindungsgemäßen Verfahrens werden Polyterpen und/oder Katalysator in einem Lösungsmittel gelöst, bevor sie miteinander in Kontakt gebracht werden. Die verwendeten Lösungsmittel sollten sich vor allem dadurch auszeichnen, dass sie die entsprechenden Terpene gut lösen können. "Gut lösen" bedeutet hierbei, dass die Konzentration des Polyterpens in der Lösung mindestens 2 Gew.-%, bevorzugt mindestens 5 Gew.-% beträgt.
Handelt es sich bei dem eingesetzten Polyterpen um Naturkautschuk oder Squalen, so sind unpolare Lösungsmittel besonders vorteilhaft. Geeignete Lösungsmittel sind beispielsweise, aber nicht erschöpfend, lineare oder cyclische Alkane wie n-Pentan, n-Hexan, n-Heptan, Cyclohexan und Cycloheptan, Aromaten wie Benzol, Hexadeuterobenzol und Toluol, Organocarbonate wie Dimethylcarbonat und Diethylcarbonat, Ether wie Diethylether und Tetrahydrofuran, Tetramethylsilan, halogenierte Kohlenwasserstoffe wie Trichlormethan, Dichlormethan und 1,2-Dichlorethan sowie perfluorierte Kohlenwasserstoffe wie Hexafluorbenzol sowie Gemische der genannten Lösungsmittel. Dem Fachmann ist bekannt, welche Lösungsmittel Naturkautschuk, Squalen und auch andere Polyterpene gut lösen. Er kann dieses Wissen anwenden, ohne den Schutzbereich der Patentansprüche zu verlassen.
Die genannten Lösungsmittel und deren Gemische eignen sich auch zum Lösen des Katalysators. Werden sowohl Polyterpen als auch Katalysator vor dem Inkontaktbringen in einem Lösungsmittel oder Lösungsmittelgemisch gelöst, so ist es vorteilhaft, aber nicht zwingend erforderlich, für beide dasselbe Lösungsmittel bzw. Lösungsmittelgemisch zu verwenden. Wird das Polyterpen in einem Lösungsmittel oder Lösungsmittelgemisch gelöst, so beträgt seine Konzentration in der Lösung 0,1 bis kleiner als 100 Gew.-%, wobei eine Lösung mit 100 Gew.-% Polyterpen dem reinen Polyterpen ohne Lösungsmittel entspricht.
Vorzugsweise wird das Polyterpen mit so viel Katalysator in Kontakt gebracht, dass pro Doppelbindung 0,001 bis 0,1 mol-% Katalysator vorliegen. Besonders vorteilhaft sind 0,01 bis 0,1 mol-% Katalysator pro Doppelbindung, in gelöster Form oder als Reinstoff.

Der Begriff "Reaktor" steht für einen abgegrenzten Raum (Behältnis, Behälter), in dem gezielte Vorgänge unter definierten Bedingungen ablaufen. "Druckfeste" Reaktoren im Sinne der vorliegenden Erfindung sind Behältnisse, die Drücken bis zu max. 100 bar standhalten. Die erforderliche Druckfestigkeit des gewählten Reaktors hängt vom gewählten Druck bei der anschließenden Reaktion mit Ethen gemäß Schritt c) ab; der Reaktor muss diesem Druck standhalten. Derartige Reaktoren sind dem Fachmann bekannt. Es kann sich beispielsweise um druckfeste Glas- oder Edelstahlreaktoren handeln.

Wird die Ethenolyse mit einer Lösung des Polyterpens durchgeführt, so muss das die Lösung vor Beginn der Ethenolyse entgast werden. Dies kann geschehen, indem bereits beim Herstellen der Lösung entgastes Lösungsmittel eingesetzt. Alternativ kann auch zuerst die Herstellung der Polyterpenlösung erfolgen und anschließend entgast werden.

Vor Beginn der Ethenolyse, d.h. bevor Ethen zugeführt und der Reaktor erwärmt wird, sollte fast kein Sauerstoff mehr im Reaktor vorhanden sein. Dies bedeutet, dass der Sauerstoffgehalt der Atmosphäre im Reaktor durch Spülen mit Schutzgas verringert werden muss. Vorteilhaft beträgt der Sauerstoffgehalt im Reaktor nach dem Spülen weniger als 1 Vol.-%. Geeignete Schutzgase sind Helium, Neon, Argon, Xenon und Stickstoff. Besonders vorteilhaft sind Argon und Stickstoff.
Optional kann das Polyterpen wie oben beschrieben in einem Lösungsmittel gelöst werden, wobei auch die Herstellung dieser Lösung unter Schutzgasatmosphäre erfolgen kann. Gleiches gilt für die optionale Herstellung einer Katalysatorlösung. Entscheidend ist die Verwendung von Schutzgas lediglich, um den Sauerstoffgehalt im Reaktor vor Beginn der Ethenolyse wie angegeben zu verringern. Zuvor kann optional Schutzgas verwendet werden, es muss vor der Ethenolyse jedoch nicht zwingend eingesetzt werden.
Nach dem Spülen mit Schutzgas wird der Reaktor mit Ethen gespült, um Reste anderer Gase zu entfernen.

Gemäß Schritt d) des erfindungsgemäßen Verfahrens wird die Ethenolyse bei einem Ethendruck von 1 bis 100 bar und einer Temperatur von 60 °C bis 150 °C für 15 min bis 24 h durchgeführt.
Der Ethendruck beträgt vorteilhaft 3 bis 20 bar. Besonders geeignet sind Reaktionstemperaturen von 100 °C bis 120 °C. Vorzugsweise wird die Ethenolyse über 1 h bis 4 h durchgeführt.
In einer besonders vorteilhaften Ausführungsform betragen der Ethendruck 3 bis 20 bar, die Reaktionstemperatur 100 °C bis 120 °C und die Reaktionszeit 1 h bis 4 h.

Eine Reaktionskontrolle ist beispielsweise mittels Gaschromatographie (GC) möglich. Nach beendeter Reaktionszeit wird die Ethenzugabe beendet, dann wird der Reaktor auf Raumtemperatur abgekühlt und ggf. auf Umgebungsdruck entspannt. Unter "Umgebungsdruck" ist dabei der der Luftdruck im den Reaktor umgebenden Raum zu verstehen.

Optional können die erhaltenen Reaktionsprodukte anschließend aufgetrennt werden, beispielsweise durch fraktionierte Destillation, durch Nanofiltration (SNRF, solvent resistant nanofiltration) oder Ultrafiltration.

Es ist festzuhalten, dass die erfindungsgemäße Ethenolyse erst bei Temperaturen größer oder gleich 50 °C und in Gegenwart von Ethen beginnt. Zweckmäßig wird die Reaktion im oben angegebenen Temperaturbereich von 60 °C bis 150 °C durchgeführt.
Die Menge des eingesetzten Katalysatorkomplexes regelt die Produktverteilung. Bei hoher Katalysatorbeladung werden alle dreifach substituierten Doppelbindungen in terminale Doppelbindungen umgewandelt. Im Falle von Naturkautschuk würde dies bedeuten, dass ausschließlich 2-Methyl-hexa-1,5-dien erzeugt wird. Um beispielsweise bevorzugt Dimere oder Trimere gemäß Anspruch 1 zu erhalten, wird die Katalysatormeng so angepasst, dass die gewünschten Produkte bevorzugt generiert werden. Der Fachmann kann die hierfür notwendige Katalysatormenge anhand seines Fachwissens leicht selbst ermitteln, ohne den Schutzbereich der Patentansprüche zu verlassen.

Besonders vorteilhaft ist die Durchführung des erfindungsgemäßen Verfahrens in einem Membranreaktor. Wenn kein Membranreaktor eingesetzt wird, liefert das erfindungsgemäße Verfahren ein Gemisch von Spaltprodukten. Will man bevorzugt Spaltprodukte innerhalb bestimmter Molekulargewichtsgrenzen erhalten, so kann man den Membranreaktor derart konfigurieren, dass Moleküle, die unterhalb dieser Grenze liegen, durch die Membran hindurchtreten und aus dem Reaktor heraus geschleust werden können. Größere Moleküle verbleiben im Reaktionsraum. Dieses schonende Verfahren gestattet es weiterhin, unzureichend gespaltene Produkte durch Zugabe von Katalysator abzubauen, so dass insgesamt eine Anreicherung der Moleküle erfolgt, die innerhalb des gewünschten Molekulargewichtsbereiches liegen.

Das erfindungsgemäße Verfahren eignet sich besonders für die Ethenolyse zum Abbau natürlich vorkommender acyclischer Polyterpene umfassend dreifach substituierte Doppelbindungen. Es ist aber auch für den Abbau anderer acyclischer Polyterpene mit dreifach substituierten Doppelbindungen geeignet.

Natürlich vorkommende acyclisch Polyterpene mit dreifach substituierten Doppelbindungen sind insbesondere Naturkautschuk und Squalen. Der Begriff "Naturkautschuk" umfasst dabei sowohl cis- als auch trans-1,4-Polyisopren (Guttapercha) natürlichen Ursprungs.
Liquid Natural Rubber wird industriell hergestellt durch den kontrollierten Abbau von Naturkautschuk mit chemischen Reagenzien (z.B. Phenylhydrazin in Gegenwart von Luftsauerstoff). Dabei enstehen Kautschukmoleküle mit verkürzter Kettenlänge. Liquid Natural Rubber-Produkte weisen Molekularmassen im Bereich von 30.000 bis 60.000 g/mol auf.

Die vorliegende Erfindung stellt erstmals niedermolekulare acyclische Terpenderivate gemäß den Formeln (II) bis (IVd) bereit. Unter "niedermolekular" werden dabei Terpenderivate mit maximal 10 Isopreneinheiten verstanden. Wird als Polyterpen Squalen eingesetzt, so umfasst dieses Edukt 6 Isopreneinheiten, und dessen Abbauprodukte umfassen zwangsläufig weniger als 6 Isopreneinheiten.
Als Derivat wird im Allgemeinen ein abgeleiteter Stoff ähnlicher Struktur zu einer entsprechenden Grundsubstanz bezeichnet. Derivate sind Stoffe, deren Moleküle an Stelle eines H-Atoms oder einer funktionellen Gruppe mit einem anderen Atom oder einer anderen Atomgruppe substituiert sind bzw. bei denen ein oder mehrere Atome/Atomgruppen entfernt wurden. In der vorliegenden Erfindung ist die Grundsubstanz ein Terpen, und die funktionelle Gruppe ist eine Isopreneinheit. Bei erfindungsgemäßen Terpenderivaten sind ein oder mehrere Wasserstoffatome oder Isopreneinheiten im Einklang mit obiger Definition durch andere Alkyl- oder Alkylengruppen substituiert.

Bei der Ethenolyse von Naturkautschuk (cis-1,4-Polyisopren) gemäß dem erfindungsgemäßen Verfahren werden Abbauprodukte mit bis zu zehn Isopreneinheiten erhalten. Die Abbauprodukte mit zwei bis sieben Isopreneinheiten sind exemplarisch gezeigt:

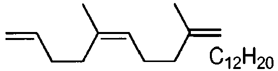

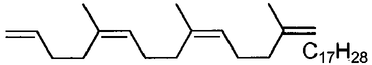

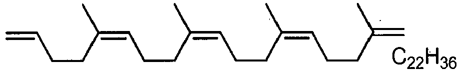

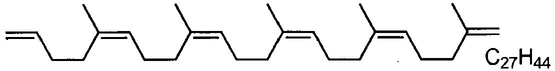

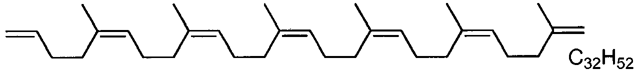

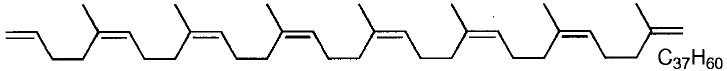

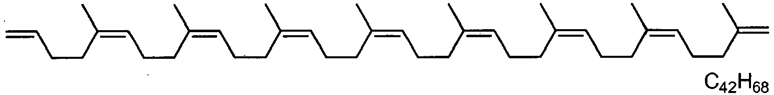

Höhere Oligomere mit den Summenformeln C₄₇H₇₆, C₅₂H₈₄, d.h. Oligomere mit bis zu zehn Isopreneinheiten, werden ebenfalls erhalten.

Bei der Ethenolyse von Guttapercha (trans-1,4-Polyisopren) gemäß dem erfindungsgemäßen Verfahren werden Abbauprodukte mit bis zu zehn Isopreneinheiten erhalten. Die Abbauprodukte mit zwei bis sechs Isopreneinheiten sind exemplarisch gezeigt:

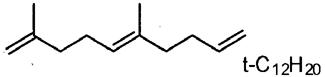

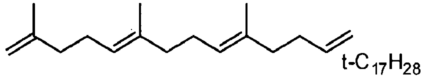

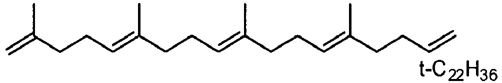

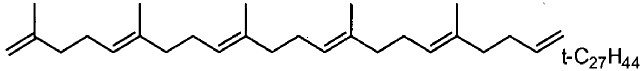

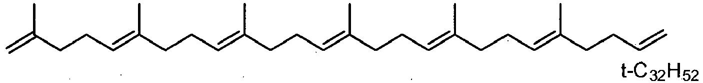

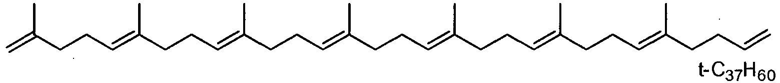

Dabei steht t für "trans".

Bei der Ethenolyse von Squalen gemäß dem erfindungsgemäßen Verfahren werden folgende Abbauprodukte erhalten:

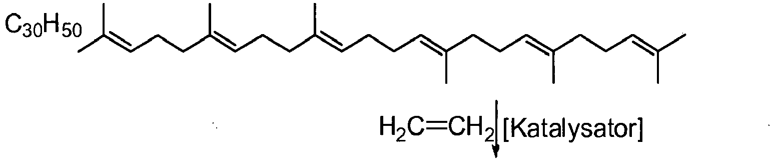

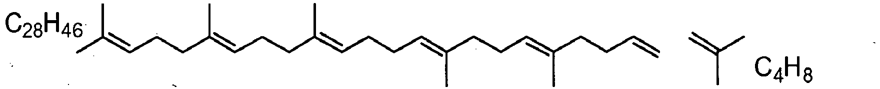

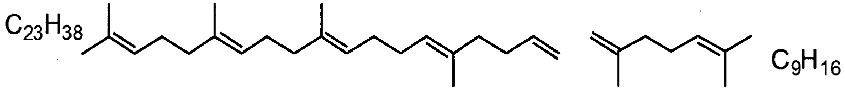

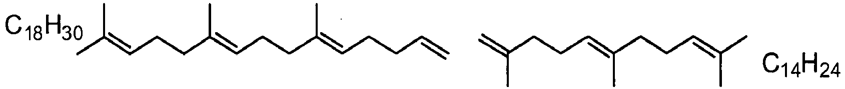

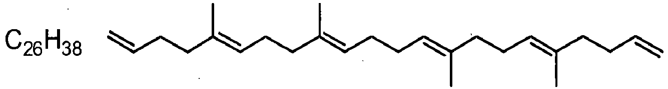

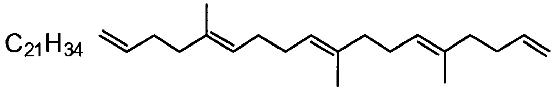

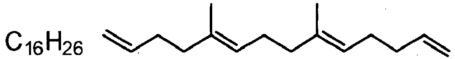

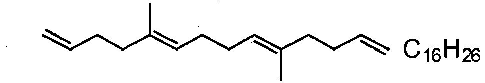

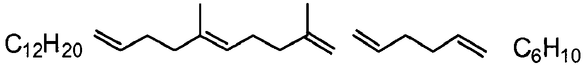

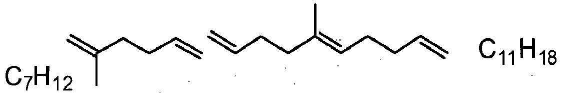

Mit Ausnahme von 2-Methylhexadien sind die gezeigten Abbauprodukte von Naturkautschuk, d.h. von natürlichem cis-1,4-Polyisopren und von natürlichem trans-1,4-Polyisopren (Guttapercha), neu und wurden bislang nicht beschrieben. Die Abbauprodukte des Squalens mit den Summenformeln C₁₆H₂₆, C₁₈H₃₀, C₂₁H₃₄ und C₂₃H₃₈ sind ebenfalls neu und bislang nicht offenbart. Das erfindungsgemäße Verfahren ermöglicht somit erstmals die Gewinnung dieser Abbauprodukte.

Bei den Abbauprodukten, die mit Hilfe des erfindungsgemäßen Verfahrens erhältlich sind, handelt es sich um Verbindungen gemäß den Formeln (II) bis (IVc), d.h. um Abbauprodukte von Naturkautschuk, Squalen oder Liquid Natural Rubber.

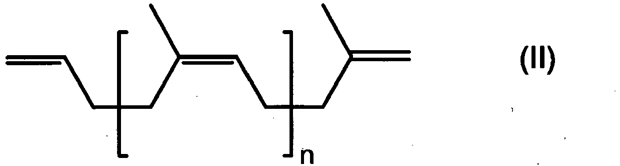

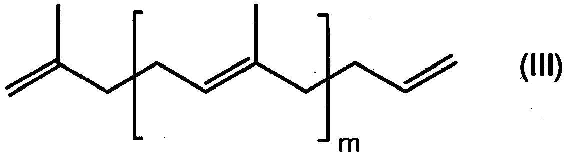

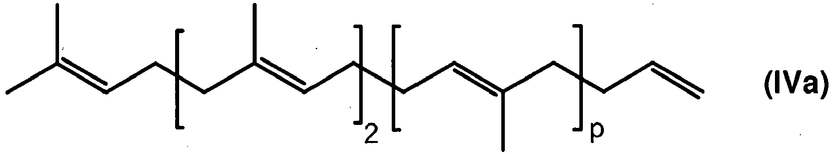

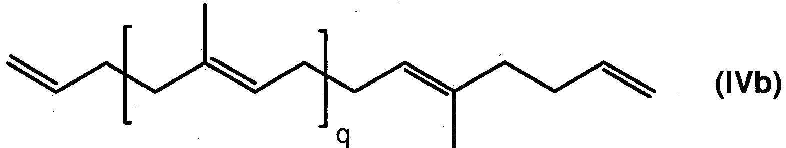

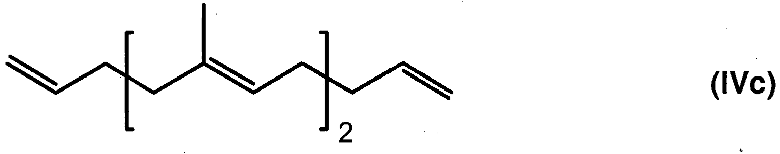

Dabei sind
n, m = 1 bis 9,
p = 0 bis 1 und
q = 1 bis 2.

Dabei handelt es sich bei Verbindungen gemäß den Formeln (II) und (III) um Abbauprodukte von Naturkautschuk, wobei Formel (II) Abbauprodukte von natürlichem cis-1,4-Polyisopren und Formel (III) um Abbauprodukte von natürlichem trans-1,4-Polyisopren (Guttapercha) darstellt. Bei den Verbindungen gemäß den Formeln (IVa), (IVb) und (IVc) handelt es sich um neue, bislang unbekannte Abbauprodukte von Squalen.

Die mit dem erfindungsgemäßen Verfahren erzeugten Abbauprodukte von Polyterpenen können zur Herstellung von Insektenpheromonen für die biologische Schädlingsbekämpfung sowie zur Herstellung von Geruchs- und Geschmacksstoffen für Parfums und kosmetische Produkte verwendet werden.

### Ausführungsbeispiele

### Allgemeine Synthesevorschrift der Ethenolyse von Squalen in Toluol (Katalysatorscreening)

In einem 250 ml Glasreaktor (Büchi miniclave) werden unter Argon-Atmosphäre Squalen (100 mg) in getrocknetem und entgastem Toluol (5 ml) gelöst. Zu dieser Lösung wird die entsprechende Menge Katalysatorkomplex (0.005 - 0.1 mol%) einer Katalysatorstammlösung (0.005 - 0.1 mol% pro mL Toluol) hinzugefügt. Der Reaktor wird 5 min mit Ethen gespült. Anschließend wird der Ethendruck innerhalb des Reaktors auf 7 bar eingestellt und der Reaktor in einem Heizbad auf die entsprechende Reaktionstemperatur (50 - 140 °C) erwärmt. Die Reaktionslösung wird mit Ethylvinylether (1 mL) versetzt und eine kleine Probe mittels Gaschromatographie analysiert.

### Allgemeine Synthesevorschrift der Ethenolyse von Squalen in Toluol (10 g Maßstab)

In einem 250 ml Glasreaktor (Büchi miniclave) werden unter Argon-Atmosphäre Squalen (10 g) in getrocknetem und entgastem Toluol (100 ml) gelöst. Zu dieser Lösung wird die entsprechende Menge Katalysatorkomplex einer Katalysatorstammlösung (0.001 - 0.1 mol% Katalysator pro mL Toluol) hinzugefügt. Der Reaktor wird ausgiebig (s.o.) mit Ethen gespült. Anschließend wird der Ethendruck innerhalb des Reaktors auf 7 bar eingestellt und der Reaktor in einem Heizbad auf die entsprechende Reaktionstemperatur (s.o.) erwärmt. Die Reaktionslösung wird mit Ethylvinylether (s.o.) versetzt. Eine kleine Probe wird mittels Gaschromatographie untersucht. Die Reaktionsprodukte werden einer fraktionierten Destillation unterworfen.

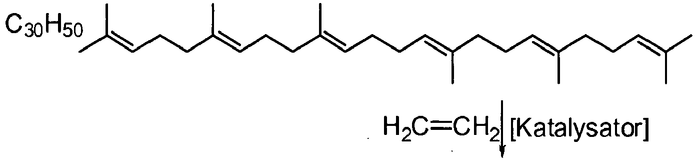

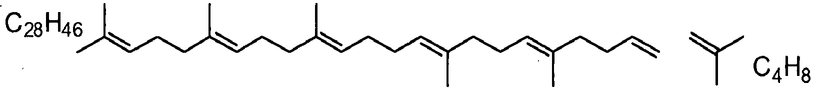

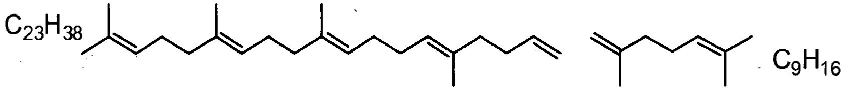

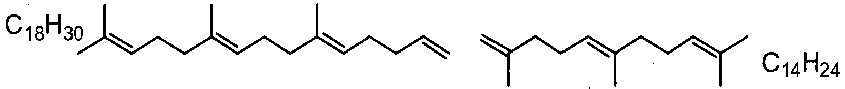

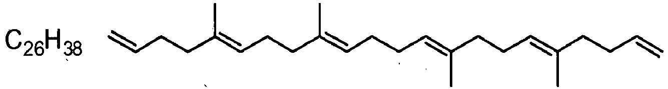

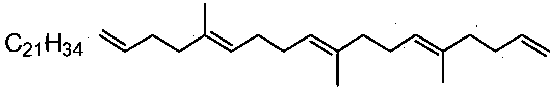

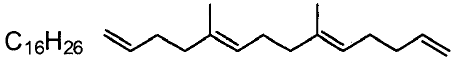

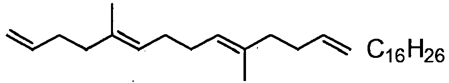

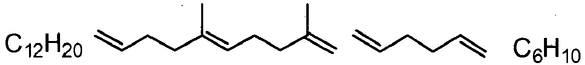

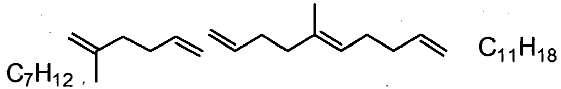

Alle Reaktionsprodukte der Squalen-Ethenolyse wurden mittels GC-MS charakterisiert.

**Tab. 1: Katalysatoroptimierung (gemäß allgemeiner Vorschrift Katalysatorscreening)**

| | Katalysator | Katalysator (mol%) | Bedingungen | Umsatz | Squalen | C₂₈H₄₆ | C₂₆H₃₈ | C₂₃H₃₈ | C₂₁H₃₄ | C₁₈H₃₀ | C₁₆H₂₆ | C₁₄H₂₄ | C₁₂H₂₀ | C₁₁H₁₈ | C₉H₁₆ |
|---|---|---|---|---|---|---|---|---|---|---|---|---|---|---|---|
| 1 | Kat. 1 | 0.1 | 100°C, 20h, C₆D₆ | 99% | - | 1059 | 1387 | 886 | 3463 | 953 | 3739 | 2373 | 7709 | 2663 | 35343 |
| 2 | Kat. 1 | 0.1 | 100°C, 6h, C₆D₆ | 99% | 720 | 3337 | 4931 | 3146 | 10688 | 3799 | 12857 | 6678 | 20352 | 7992 | 7387 |
| 3 | Kat. 1 | 0.05 | 100°C, 6h, C₆D₆ | 82% | 18837 | 20904 | 5893 | 10772 | 7552 | 10395 | 7725 | 11880 | 8192 | 3007 | 9600 |
| 4 | Kat. 1 | 0.01 | 100°C, 3h, C₆D₆ | 33% | 66350 | 16680 | 1052 | 5854 | 787 | 4821 | 720 | 4351 | 597 | 196 | 3050 |
| 5 | Kat. 1 | 0.01 | 100°C, 1h, C₆D₆ | 36% | 43561 | 12554 | 850 | 4247 | 663 | 3493 | 596 | 3100 | 467 | 147 | 2111 |
| 6 | Kat. 1 | 0.01 | 120°C, 3h, C₆D₆ | 61% | 16376 | 10229 | 1527 | 3715 | 1267 | 3237 | 1156 | 3202 | 1050 | 317 | 1885 |
| 7 | Kat. 1 | 0.01 | 120°C, 1h, C₆D₆ | 29% | 66230 | 14612 | 756 | 5041 | 556 | 4080 | 480 | 3577 | 390 | - | 2449 |
| 8 | Kat. 1 | 0.01 | 120°C, 3h, Toluol | 63% | 28165 | 19423 | 3410 | 7188 | 2660 | 6091 | 2311 | 5894 | 2055 | 641 | 3858 |
| 9 | Kat. 1 | 0.005 | 120°C, 3h, Toluol | 22% | 76900 | 12226 | 463 | 4022 | 338 | 3186 | 295 | 2811 | 225 | - | 1800 |
| 10 | Kat. 1 | 0.005 | 140°C, 3h, Toluol | 12% | 25079 | 2210 | - | 722 | - | 511 | - | 450 | - | - | - |
| 11 | Kat. 1 | 0.005 | 120°C, 3h, Toluol,8 Gew.% Squalen | 35% | 53057 | 14933 | 961 | 4770 | 694 | 3803 | 597 | 3357 | 482 | 139 | 2144 |
| 12 | Kat. 1 | 0.005 | 120°C, 3h, CH₂Cl₂ | 3% | 84285 | 1738 | - | 657 | - | 324 | - | 214 | - | - | - |
| 13 | Kat. 1 | 0.005 | 120°C, 3h, Pentan | 5% | 47450 | 1437 | - | 580 | - | 312 | - | 267 | - | - | - |
| 14 | Kat. 1 | 0.005 | 120°C, 3h, Cyclohexan | <1% | - | - | - | - | - | - | - | - | - | - | - |
| 15 | Kat. 5 | 0.005 | 120°C, 3h, Toluol | 2% | 89300 | 1601 | - | 631 | - | - | - | - | - | - | - |
| 16 | Kat. 3 | 0.005 | 120°C, 3h, Toluol | 3% | 85475 | 1664 | - | 710 | - | - | - | - | - | - | - |
| 17 | Kat. 3 | 0.01 | 120°C, 3h, Toluol | 28% | 60878 | 12912 | 685 | 4199 | 490 | 3332 | 384 | 2834 | 318 | 81 | 1837 |
| 18 | Kat. 6 | 0.005 | 120°C, 3h, Toluol | <1% | - | - | - | - | - | - | - | - | - | - | - |
| 19 | Kat. 2 | 0.01 | 120°C, 3h, Toluol | 77% | 30879 | 33561 | 9174 | 12659 | 7609 | 10847 | 6306 | 11282 | 5362 | 1504 | 6284 |
| 20 | Kat. 4 | 0.01 | 120°C, 3h, Toluol | 50% | 65000 | 30633 | 3424 | 10519 | 2851 | 8059 | 2110 | 8719 | 1901 | 469 | 3766 |
| 21 | Kat. 8 | 0.01 | 120°C, 3h, Toluol | 30% | 76827 | 18145 | 1030 | 5740 | 700 | 4651 | 614 | 4069 | 474 | 135 | 2499 |
| 22 | Kat. 8 | 0.005 | 120°C, 3h, Toluol | 8% | 108480 | 6090 | 50 | 1943 | - | 1414 | - | 1250 | - | - | - |
| 23 | Kat. 7 | 0.005 | 120°C, 3h, Toluol | 6% | 63440 | 1826 | 95 | 885 | 364 | 446 | - | 345 | - | - | - |
| 24 | Kat. 9 | 0.01 | 120°C, 3h, Toluol | 53% | 52145 | 26556 | 3401 | 9075 | 2780 | 7091 | 2106 | 7589 | 1837 | 504 | 3479 |
| 25 | Kat. 10 Vergleichskatalysator | 0.005 | 120°C, 3h, Toluol | <1% | - | - | - | - | - | - | - | - | - | - | - |
| 26 | Kat. 10 Vergleichskatalysator | 0.005 | 140°C, 3h, Toluol | 3% | 92401 | 1602 | - | 632 | 130 | 327 | - | 237 | - | - | 152 |
| 27 | Kat. 11 Vergleichskatalysator | 0.01 | 120°C, 3h, Toluol | <1% | | | | | | | | | | | |
| 28 | Kat. 12 Vergleichskatalysator | 0.005 | 120°C, 3h, Toluol | 15% | 81633 | 16173 | 745 | 5807 | 622 | 4591 | 557 | 4076 | 421 | 151 | 2508 |

Wenn nicht anders gekennzeichnet, wurde eine Squalen-Lösung (5 Gew.%) in einem Lösungsmittel verwendet. Die Zahlen in den Spalten unter den einzelnen Abbauprodukten stehen für die Integrale der jeweiligen Substanzpeaks in den Gaschromatogrammen.

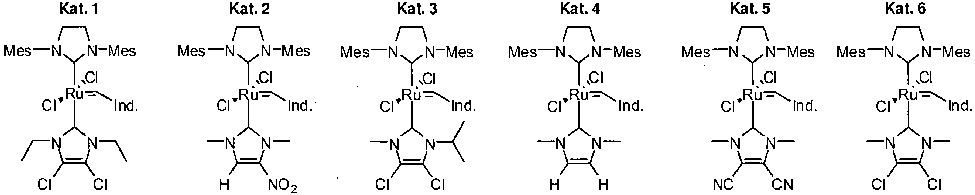

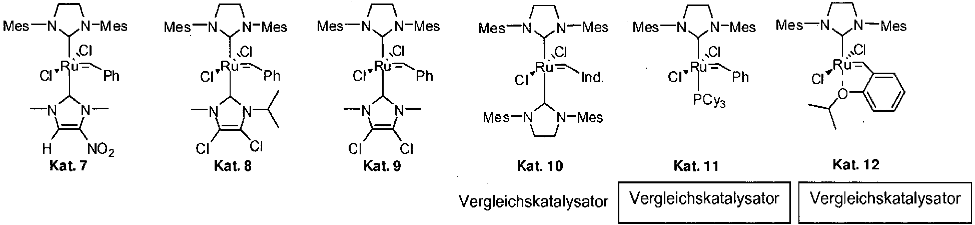

Mes = Mesitylen = 2,4,6-Trimethylphenylrest
Ind = 3-Phenylindenylid-1-en-Rest
Bei Katalysator 11 handelt es sich um eine Verbindung von Typ Grubbs II und bei Katalysator 12 um einen Grubbs-Hoveyda 11-Katalysator. Diese beiden Katalysatoren dienten als Vergleichssubstanzen.

### Allgemeine Synthesevorschrift der Ethenolyse von Naturkautschuk (Katalysatorscreening)

In einem 250 ml Glasreaktor (Büchi miniclave) werden unter Argon-Atmosphäre Naturkautschuk (50 mg) in getrocknetem und entgastem Hexadeuterobenzol (5 ml) gelöst. Zu dieser Lösung wird die entsprechende Menge Katalysatorkomplex einer Katalysatorstammlösung (s.o.) in Hexadeuterobenzol hinzugefügt. Der Reaktor wird ausgiebig (s.o.) mit Ethen gespült. Anschließend wird der Ethendruck innerhalb des Reaktors auf 7 bar eingestellt und der Reaktor in einem Heizbad auf die entsprechende Reaktionstemperatur (s.o.) erwärmt. Die Zusammensetzung der Reaktionslösung wird mittels ¹H NMR Spektroskopie analysiert.

### Allgemeine Synthesevorschrift der Ethenolyse von Naturkautschuk (10 g Maßstab)

In einem 250 ml Glasreaktor (Büchi miniclave) werden unter Argon-Atmosphäre Naturkautschuk (10 g) in getrocknetem und entgastem Toluol (100 ml) gelöst. Zu dieser Lösung wird die entsprechende Menge Katalysatorkomplex einer Katalysatorstammlösung (s.o.) in Toluol hinzugefügt. Der Reaktor wird ausgiebig (s.o.) mit Ethen gespült. Anschließend wird der Ethendruck innerhalb des Reaktors auf 7 bar eingestellt und der Reaktor in einem Heizbad auf die entsprechende Reaktionstemperatur (s.o.) erwärmt. Die Reaktionslösung wird nach erfolgter Reaktion mit Ethylvinylether versetzt. Die verschiedenen Spaltprodukte werden mittels fraktionierter Destillation aufgetrennt.

### Einige Abbauprodukte von Hevea brasiliensis-Naturkautschuk (beispielhaft) Ethenolyse von Naturkautschuk

In einem 100 ml Schlenkkolben werden unter Argonatmosphäre Naturkautschuk (4 g) in Chlorofom, abs. (45 ml) gelöst. In einem 250 ml Glasreaktor (Büchi miniclave) werden unter Argon die LNR-Lösung sowie Katalysator 2 (siehe Tabelle 1) (54.0 mg, 0.059 mmol, 0.1 mol% pro Doppelbindungen) vereinigt. Der Reaktor wird 5 min mit Ethen gespült. Anschließend wird der Ethendruck innerhalb des Reaktors auf 7 bar eingestellt und der Reaktor in einem Ölbad für 3h auf 120°C erhitzt. Der Katalysator wird nach erfolgter Reaktion durch Zugabe von Ethylvinylether (1 mL) deaktiviert. Das Reaktionsgemisch wird mit Chloroform auf 200 ml aufgefüllt, diese Lösung wird in eine Millipore Zelle überführt und einer Nanofiltration (Membran: MWCO (Molekulargewichtsausschluß 500 Dalton, Fluss: 1ml/min, Δp = 3 bar) unterworfen. Es werden zunächst 150 ml abfiltriert, anschließend die Lösung erneut auf 200 ml aufgefüllt und erneut 150 ml abfiltriert. Das Filtrat wird am Rotationsverdampfer auf ungefähr 5 mL eingeengt. Aus dem Rückstand werden die einzelnen Spaltprodukte säulenchromatographisch (Eluent: Pentan oder Cyclohexan) oder destillativ (Vakuumdestillation) in reiner Form gewonnen. (Charakterisierungsdaten siehe Vorschrift LNR)

### Ethenolyse von LNR (Liquid Natural Rubber)

In einem 100 ml Schlenkkolben werden unter Argonatmosphäre Liquid Natural Rubber Naturkautschuk (4 g) in Chlorofom, abs. (45 ml) gelöst. In einem 250 ml Glasreaktor (Büchi miniclave) werden unter Argon die LNR-Lösung sowie Katalysator 1 (siehe Tabelle 1) (50.9 mg, 0.059 mmol, 0.1 mol% pro Doppelbindungen) vereinigt. Der Reaktor wird 5 min mit Ethen gespült. Anschließend wird der Ethendruck innerhalb des Reaktors auf 7 bar eingestellt und der Reaktor in einem Ölbad für 3h auf 120°C erhitzt. Der Katalysator wird nach erfolgter Reaktion durch Zugabe von Ethylvinylether (1 mL) deaktiviert.
Das Reaktionsgemisch wird mit Chlorofom auf 200 ml aufgefüllt, diese Lösung wird in eine Millipore Zelle überführt und einer Nanofiltration (Membran: MWCO (Molekulargewichtsausschluß 500 Dalton, Fluss: 1ml/min, Δp = 3 bar) unterworfen. Es werden zunächst 150 ml abfiltriert, anschließend die Lösung erneut auf 200 ml aufgefüllt und erneut 150 ml abfiltriert. Das Filtrat wird am Rotationsverdampfer auf ungefähr 5 mL eingeengt. Aus dem Rückstand werden die einzelnen Spaltprodukte säulenchromatographisch (Eluent: Pentan oder Cyclohexan) oder destillativ (Vakuumdestillation) in reiner Form gewonnen.

### Charakterisierungsdaten einiger Ethenolyseprodukte

### 2,6-Dimethyl-deca-1,5,9-trien

¹H NMR (500 MHz, CDCl₃) δ 5.90 - 5.78 (m, 1 H *(9)*), 5.15 (t (13 Hz), m, 1 H *(5)*), 5.02 (d(17 Hz), m, 1 H *(10)*), 4.95 (d(13 Hz),m, 1 H *(10)*), 4.71 (d (13 Hz),m, 2H *(1)*), 2.15 - 2.00 (m, 8 H *(3,4,7,8-CH2)*), 1.72 (s, 3H *(11)*), 1.69 (m, 3H *(12)*).
¹³C NMR (126 MHz, CDCl₃) δ 145.98, 138.85, 134.98, 125.25, 114.53, 109.98, 38.24, 32.41, 31.50, 26.25, 23.47, 22.60.
Reinheit (HPLC) > 95%

### 2,6,10-Trimethyl-tetradeca-1,5,9,13-tetraen

¹H NMR (500 MHz, CDCl₃) δ 5.89 - 5.76 (m, 1 H *(13)*), 5.19 - 5.10 (m, 2H *(5,9)*), 5.02 (d(17 Hz), m, 1 H *(14)*), 4.95 (d(13 Hz),m, 1 H *(14)*), 4.70 (d (13 Hz),m, 2H *(1)*), 2.17 - 2.00 (m, 12H *(3,4,7,8,11,12-CH2)*), 1.73 (s, 3H *(15)*), 1.70 (m, 3H, 16), 1.69 (m, 3H *(17)*).
¹³C NMR (126 MHz, CDCl₃) δ 145.98, 138.80, 135.37, 134.93, 125.48, 125.07, 114.54, 109.93, 38.26, 32.48, 32.41, 31.46, 26.53, 26.30, 23.56, 23.49, 22.64.
Reinheit (HPLC) > 95%

### 2,6,10,14-Tetramethyl-octadeca-1,5,9,13,17-pentaen

¹H NMR (500 MHz, CDCl₃) δ 5.86 - 5.77 (m, 1 H *(17)*), 5.18 - 5.10 (m, 3H *(5,9,13)*), 5.03 (d 17 Hz), m, 1 H *(18)*), 4.95 (d(13 Hz)m, 1 H *(18)*), 4.70 (d 13 Hz), m, 2H *(1)*), 2.15 - 2.01 (m, 16H *(3,4,7,8,11,12,15,16)*), 1.72 (s, 3H *(19)*), 1.70 - 1.67 (m, 9H *(20,21,22)*).
¹³C NMR (126 MHz, CDCl₃) δ 145.99, 138.81, 135.41, 135.38, 134.92, 125.46, 125.27, 125.03, 114.54, 109.94, 38.26, 32.48, 32.41, 32.36, 31.48, 26.59, 26.53, 26.29, 23.58, 23.56, 23.49, 22.65.
Reinheit (HPLC) > 90%

### 2,6,10,14,18-Pentamethyl-docosa-1,5,9,13,17,21-hexaen

¹H NMR (500 MHz, CDCl₃) δ 5.87 - 5.76 (m, 1H *(21)*), 5.19 - 5.10 (m, 4H *(5,9,13,17)*), 5.03 (d (17 Hz), m, 1 H *(22)*), 4.95 (d(13 Hz), m, 1 H *(22)*), 4.70 (d 813 Hz), m, 2H *(1)*), 2.16 - 2.01 (m, 20H *(3,4,7,8,11,12,15,16,19,20)*), 1.73 (s, 3H *(23)*), 1.69 (s, 12H *(24,25,26,27)*).
¹³C NMR (126 MHz, CDCl₃) δ 145.98, 138.80, 135.40, 135.34, 134.91, 125.46, 125.26, 125.22, 125.03, 114.54, 109.94, 38.26, 32.48, 32.41, 32.36, 31.48, 26.59, 26.58, 26.52, 26.28, 23.58, 23.56, 23.50, 22.65.
Reinheit (HPLC) > 90%

## Patentansprüche

1. Verfahren zur Ethenolyse von acyclischen Polyterpenen, die dreifach substituierte Doppelbindungen umfassen, umfassend die Schritte:
a) Inkontaktbringen eines Polyterpens mit einem Katalysatorkomplex ausgewählt aus [(NHC)(NHC_{ewg})MeX₂(CHPh)] und [(NHC)(NHC_{ewg})MeX₂(3-phenylindenylid-1-en)] in einem druckfesten Reaktor, worin
• NHC und NHC_{ewg} für *N*-heterocyclische Carbenliganden stehen, wobei NHC elektronenreicher als NHC_{ewg} ist,
• Me ausgewählt ist aus Ru und Os,
• X ausgewählt ist aus F, Cl, Br, I, CN, SCN, OCN, NCO,
b) Spülen des Reaktors mit Schutzgas,
c) Spülen des Reaktors mit Ethen,
d) Reaktion des Gemisches aus Polyterpen und Katalysatorkomplex mit Ethen bei einem Ethendruck von 1 bis 100 bar und einer Temperatur von 60 °C bis 150 °C für 15 min bis 24 h,
e) Beenden der Ethenzugabe, Abkühlen auf Raumtemperatur und ggf. Entspannen auf Umgebungsdruck,
f) optional Auftrennen der erhaltenen Spaltprodukte.

2. Verfahren gemäß Anspruch 1, **dadurch gekennzeichnet, dass** der Katalysatorkomplex ausgewählt ist aus Verbindungen gemäß Formel (I): 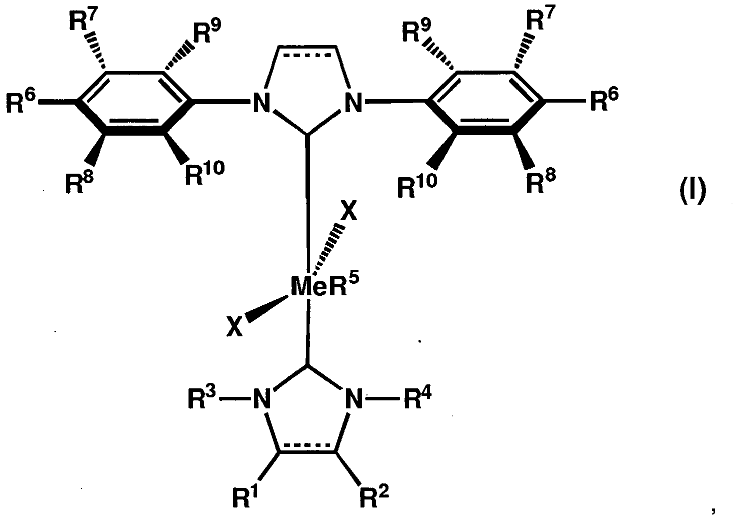 worin
R¹ und R² unabhängig voneinander für -H, -F, -Cl, -Br, -I, -CN, -SCN, -OCN, -NCO, -NO₂ oder eine lineare, verzweigte oder cyclische Alkylgruppe mit 1 bis 20 Kohlenstoffatomen, stehen,
R³ und R⁴ unabhängig voneinander für -H, eine lineare, verzweigte oder cyclische Alkylgruppe mit 1 bis 20 Kohlenstoffatomen, oder eine Gruppe ausgewählt aus 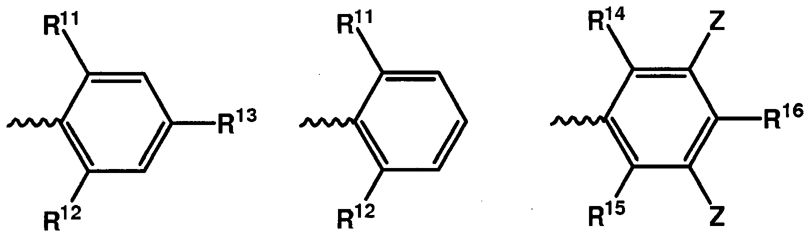 stehen, worin
R¹¹, R¹² und R¹³ unabhängig voneinander für -H, -F, -Cl, -Br, -I, -CN, -SCN, -OCN, -NCO, -NO₂ oder eine lineare oder verzweigte Alkylgruppe mit 1 bis 20 Kohlenstoffatomen stehen,
R¹⁴, R¹⁵ und R¹⁶ unabhängig voneinander für -H oder eine lineare oder verzweigte Alkylgruppe mit 1 bis 20 Kohlenstoffatomen stehen
und
Z für -NO₂ oder -SO₃H stehen,
R⁵ für eine Gruppe 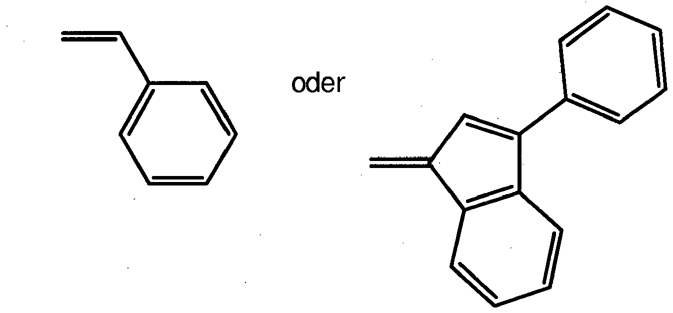 steht,
R⁶ für -H oder eine lineare, verzweigte oder cyclische Alkylgruppe mit 1 bis 20 Kohlenstoffatomen und
R⁷ und R⁸ unabhängig voneinander für -H, -F, -Cl, -Br, -I, -CN, -SCN, -OCN, -NCO, -NO₂, -OH, -SH, -NH₂, -OCH₃ oder eine lineare, verzweigte oder cyclische Alkylgruppe mit 1 bis 20 Kohlenstoffatomen und
R⁹ und R¹⁰ unabhängig voneinander für eine lineare oder verzweigte Alkylgruppe mit 1 bis 4 Kohlenstoffatomen stehen und
Me für Ru oder Os steht,
X für ein Halogen oder Pseudohalogen steht, ausgewählt aus -F, -Cl, -Br, -I, -CN, -SCN, -OCN, -NCO
und 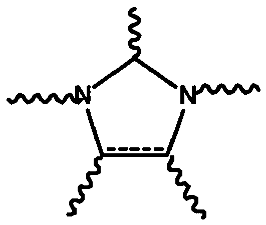 für einen Imidazolidin- oder 2,3-Dihydroimidazolrest steht.

3. Verfahren gemäß einem der Ansprüche 1 und 2, **dadurch gekennzeichnet, dass** das Polyterpen ausgewählt ist aus Naturkautschuk, Liquid Natural Rubber und Squalen.

4. Verfahren gemäß einem der Ansprüche 1 bis 3, **dadurch gekennzeichnet, dass** es sich beim Zentralatom Me des Katalysatorkomplexes um Ruthenium handelt.

5. Verfahren gemäß einem der Ansprüche 1 bis 4, **dadurch gekennzeichnet, dass** der Rest R⁶ ein Wasserstoffatom oder eine Methylgruppe ist, die Reste R⁷ und R⁸ Wasserstoffatome sind und die Reste R⁹ und R¹⁰ Methylgruppen darstellen.

6. Verfahren gemäß einem der Ansprüche 1 bis 5, **dadurch gekennzeichnet, dass** die Reste R¹ und R² unabhängig voneinander ausgewählt sind aus -H, -Cl, -NO₂, -CN und 3,5-Dinitro-2,4,6-trimethylphenyl.

7. Verfahren gemäß einem der Ansprüche 1 bis 6, **dadurch gekennzeichnet, dass** die Reste R³ und R⁴ unabhängig voneinander ausgewählt sind aus Methyl-, Ethyl-und Isopropylgruppen.

8. Verfahren gemäß einem der Ansprüche 1 bis 7, **dadurch gekennzeichnet, dass** pro Doppelbindung des Polyterpens 0,01 bis 0,1 mol-% des Katalysatorkomplexes eingesetzt werden.

9. Verfahren gemäß einem der Ansprüche 1 bis 8, **dadurch gekennzeichnet, dass** der Ethendruck 3 bis 20 bar, die Reaktionstemperatur 100 °C bis 120 °C und die Reaktionszeit 1 h bis 4 h betragen.

## Claims

1. Method for the ethenolysis of acyclic polyterpenes which comprise trisubstituted double bonds, comprising the steps of:
a) contacting a polyterpene with a catalyst complex selected from [(NHC)(NHC_{ewg})MeX₂(CHPh)] and [(NHC)(NHC_{ewg})MeX₂(3-phenylindenylid-1-en)] in a pressure-resistant reactor, in which
• NHC and NHC_{ewg} stand for N-heterocyclic carbene ligands,
wherein NHC is electron-richer than NHC_{ewg},
• Me is selected from Ru and Os,
• X is selected from F, Cl, Br, I, CN, SCN, OCN, NCO,
b) flushing the reactor with protective gas,
c) flushing the reactor with ethene,
d) reacting the polyterpene and catalyst complex mixture with ethene at an ethene pressure of from 1 to 100 bar and a temperature of from 60 °C to 150 °C for from 15 minutes to 24 hours,
e) ending the ethene addition, cooling to room temperature and if necessary relieving to ambient pressure,
f) optionally separating off the obtained cleavage products.

2. Method according to claim 1, **characterised in that** the catalyst complex is selected from compounds according to formula (I): 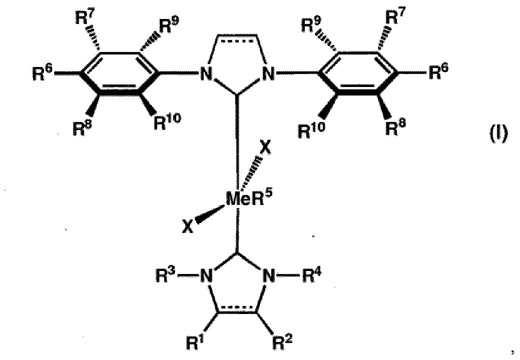 in which
R¹ and R² stand, independently of each other, for -H, -F, -Cl, -Br, -I, -CN, -SCN, -OCN, -NCO, -NO₂ or a linear, branched or cyclic alkyl group having from 1 to 20 carbon atoms,
R³ and R⁴ stand, independently of each other, for -H, a linear, branched or cyclic alkyl group having from 1 to 20 carbon atoms, or a group selected from 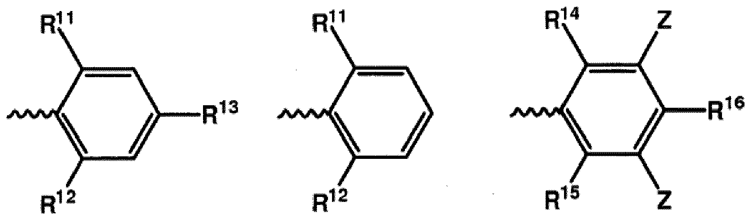
in which
R¹¹, R¹² and R¹³ stand, independently of each other, for -H, -F, -Cl, -Br, - I, -CN, -SCN, -OCN, -NCO, -NO₂ or a linear or branched alkyl group having from 1 to 20 carbon atoms,
R¹⁴, R¹⁵ and R¹⁶ stand, independently of each other, for -H or a linear or branched alkyl group having from 1 to 20 carbon atoms,
and
Z stands for -NO₂ or -SO₃H,
R⁵ stands for a group having the form 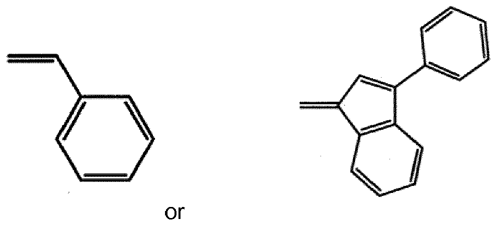
R⁶ stands for-H or a linear, branched or cyclic alkyl group having from 1 to 20 carbon atoms, and
R⁷ and R⁸ stand, independently of each other, for -H, -F, -Cl, -Br, -I, -CN, -SCN, -OCN, -NCO, -NO₂, -OH, -SH, -NH₂, -OCH₃ or a linear, branched or cyclic alkyl group having from 1 to 20 carbon atoms, and
R⁹ and R¹⁰ stand, independently of each other, for a linear or branched alkyl group having from 1 to 4 carbon atoms, and
Me stands for Ru or Os,
X stands for a halogen or pseudohalogen selected from -F, -Cl, -Br, -I,
-CN, -SCN, -OCN, -NCO,
and 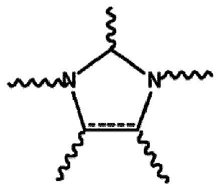 stands for an imidazolidine- or 2,3-dihydroimidazole functional group.

3. Method according to either claim 1 or claim 2, **characterised in that** the polyterpene is selected from natural rubber, liquid natural rubber and squalene.

4. Method according to any of claims 1 to 3, **characterised in that** the central atom Me of the catalyst complex is ruthenium.

5. Method according to any of claims 1 to 4, **characterised in that** the functional group R⁶ is a hydrogen atom or a methyl group, the functional groups R⁷ and R⁸ are hydrogen atoms and the functional groups R⁹ and R¹⁰ represent methyl groups.

6. Method according to any of claims 1 to 5, **characterised in that** the functional groups R¹ and R² are selected, independently of each other, from -H, -Cl, -NO₂, -CN and 3,5-dinitro-(2,4,6-trimethylphenyl).

7. Method according to any of claims 1 to 6, **characterised in that** the functional groups R³ and R⁴ are selected, independently of each other, from methyl, ethyl and isopropyl groups.

8. Method according to any of claims 1 to 7, **characterised in that** from 0.01 to 0.1 mol.% of the catalyst complex is applied per double bond of the polyterpene.

9. Method according to any of claims 1 to 8, **characterised in that** the ethene pressure is from 3 to 20 bar, the reaction temperature is from 100 °C to 120 °C and the reaction time is from 1 hour to 4 hours.

## Revendications

1. Procédé d'éthénolyse de polyterpènes acycliques, qui comprennent des doubles liaisons substituées trois fois, comprenant les étapes consistant à :
a) mettre en contact un polyterpène avec un complexe catalytique choisi parmi [(NHC) (NHC_{ewg}) MeX₂ (CHPh)] et [(NHC) (NHC_{ewg})MeX₂ (3-phénylindénylid-1-ène)] dans un réacteur résistant à la pression, dans lequel :
• NHC et NHC_{ewg} désignent des ligands carbènes N-hétérocycliques, où NHC est plus riche en électrons que NHC_{ewg},
• Me est choisi parmi Ru et Os, et
• X est choisi parmi F, Cl, Br, I, CN, SCN, OCN et NCO,
b) rincer le réacteur avec un gaz de protection,
c) rincer le réacteur avec de l'éthène,
d) faire réagir le mélange de polyterpène et de complexe catalytique avec de l'éthène sous une pression de l'éthène de 1 à 100 bars et à une température de 60 à 150 °C pendant 15 min à 24 h,
e) mettre fin à l'addition d'éthène, refroidir à température ambiante et éventuellement détendre à la pression ambiante, et
f) séparer éventuellement les produits de séparation obtenus.

2. Procédé selon la revendication 1, **caractérisé en ce que** le complexe catalytique est choisi parmi les composés de formule (I) : 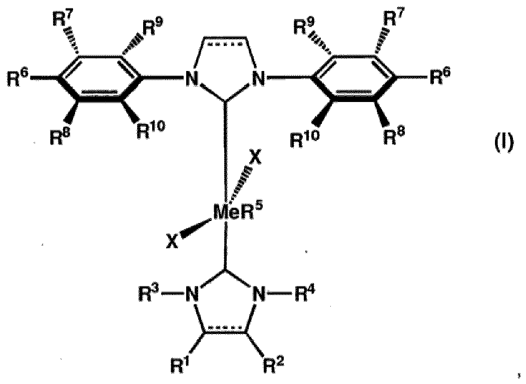 dans laquelle :
R¹ et R² représentent, indépendamment l'un de l'autre, -H, -F, -Cl, -Br, -I, -CN, -SCN, -OCN, -NCO, -NO₂ ou un groupement alkyle linéaire, ramifié ou cyclique avec 1 à 20 atomes de carbone,
R³ et R⁴ représentent, indépendamment l'un de l'autre, -H, un groupement alkyle linéaire, ramifié ou cyclique avec 1 à 20 atomes de carbone ou un groupement choisi parmi les suivants : 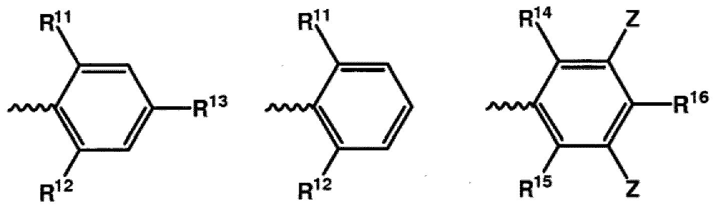 dans lesquels :
R¹¹, R¹² et R¹³ représentent, indépendamment l'un de l'autre, -H, -F, -Cl, -Br, -I, -CN, -SCN, -OCN, -NCO, -NO₂ ou un groupement alkyle linéaire ou ramifié avec 1 à 20 atomes de carbone,
R¹⁴, R¹⁵ et R¹⁶ représentent, indépendamment l'un de l'autre, -H ou un groupement alkyle linéaire ou ramifié avec 1 à 20 atomes de carbone, et
Z représente -NO₂ ou -SO₃H,
R⁵ représente un groupement : 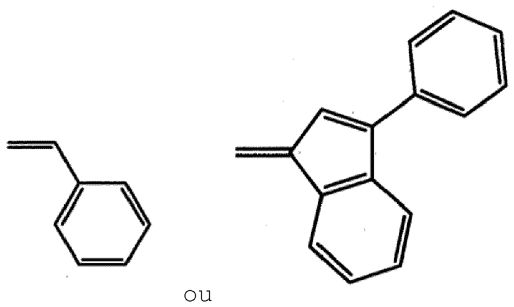
R⁶ représente -H ou un groupement alkyle linéaire, ramifié ou cyclique avec 1 à 20 atomes de carbone et
R⁷ et R⁸ représentent, indépendamment l'un de l'autre, -H, -F, -Cl, -Br, -I, -CN, -SCN, -OCN, -NCO, -NO₂, -OH, -SH, -NH₂, -OCH₃ ou un groupement alkyle linéaire, ramifié ou cyclique avec 1 à 20 atomes de carbone,
R⁹ et R¹⁰ représentent, indépendamment l'un de l'autre, un groupement alkyle linéaire ou ramifié avec 1 à 4 atomes de carbone, et
Me représente Ru ou Os,
X représente un halogène ou un pseudo-halogène choisi parmi -F, -Cl, -Br, -I, -CN, -SCN, -OCN, -NCO et 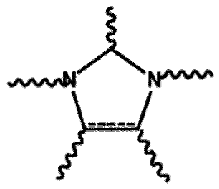 représente un résidu d'imidazolidine ou de 2,2-dihydroimidazol.

3. Procédé selon l'une quelconque des revendications 1 et 2, **caractérisé en ce que** le polyterpène est choisi parmi le caoutchouc naturel, le caoutchouc naturel liquide et le squalène.

4. Procédé selon l'une quelconque des revendications 1 à 3, **caractérisé en ce que** l'atome central Me du complexe catalytique est le ruthénium.

5. Procédé selon l'une quelconque des revendications 1 à 4, **caractérisé en ce que** le résidu R⁶ est un atome d'hydrogène ou un groupement méthyle, les résidus R⁷ et R⁸ sont des atomes d'hydrogène et les résidus R⁹ et R¹⁰ représentent des groupements méthyle.

6. Procédé selon l'une quelconque des revendications 1 à 5, **caractérisé en ce que** les résidus R¹ et R² sont choisis, indépendamment l'un de l'autre, parmi -H, -Cl, -NO₂, -CN et 3,5-dinitro-2,4,6-triméthylphényle.

7. Procédé selon l'une quelconque des revendications 1 à 6, **caractérisé en ce que** les résidus R³ et R⁴ sont choisis, indépendamment l'un de l'autre, parmi les groupements méthyle, éthyle et isopropyle.

8. Procédé selon l'une quelconque des revendications 1 à 7, **caractérisé en ce que** l'on utilise 0,01 à 0,1 % en mole du complexe catalytique par double liaison du polyterpène.

9. Procédé selon l'une quelconque des revendications 1 à 8, **caractérisé en ce que** la pression de l'éthène atteint 3 à 20 bars, la température réactionnelle 100 à 120 °C et la durée de la réaction 1 à 4 h.
